# EUROPÄISCHE PATENTSCHRIFT

(11) **EP 1 096 998 B1**
(45) Veröffentlichungstag und Bekanntmachung des Hinweises auf die Patenterteilung: **19.03.2003**
(21) Anmeldenummer: 99936358.3
(22) Anmeldetag: 29.05.1999
(51) Int. Cl.: B01L 3/00, B01L 7/00

(54) **VERFAHREN UND VORRICHTUNG ZUR PROBENVORBEREITUNG FÜR DEN NACHWEIS EINER NUKLEOTIDSEQUENZ**
METHOD AND DEVICE FOR PREPARING SAMPLES FOR DETECTING A NUCLEOTIDE SEQUENCE
PROCEDE ET DISPOSITIF POUR PREPARER DES ECHANTILLONS DESTINES A LA DETECTION D'UNE SEQUENCE NUCLEOTIDIQUE

(30) Priorität: 12.06.1998 DE 19826153
(43) Veröffentlichungstag der Anmeldung: 09.05.2001
(73) Patentinhaber: november Aktiengesellschaft Gesellschaft für Molekulare Medizin, 91056 Erlangen (DE)
(72) Erfinder: BERTLING, Wolf, D-91056 Erlangen (DE)
(74) Vertreter: Gassner, Wolfgang, Dr.
(86) Internationale Anmeldenummer: DE9901589
(87) Internationale Veröffentlichungsnummer: WO99064157

(56) Entgegenhaltungen:
- WO-A-96/02836
- WO-A-98/25701
- FR-A- 2 672 301
- GB-A- 2 333 250
- US-A- 5 484 734
- US-A- 5 556 773
- US-A- 5 585 242
- US-A- 5 604 130

## Beschreibung

Die Erfindung betrifft ein Verfahren zur Probenvorbereitung für den Nachweis einer Nukleotidsequenz mittels Polymerase-Ketten-Reaktion (PCR). Sie betrifft ferner eine Vorrichtung nach dem Oberbegriff des Anspruchs 14.

Aus der US 5,455,175 ist ein so genannter Thermocycler bekannt, mit dem eine Mehrzahl an flüssigen biologischen Proben zur Durchführung der PCR wiederkehrend einem vorgegebenen Temperaturprofil ausgesetzt werden kann. Um die erforderliche Zeit für die Temperaturbehandlung zu verkürzen, ist hier jeweils ein kleines Volumen der biologischen Proben in einer dünnwandigen Glaskapillare aufgenommen. Dazu muss jede Probe einzeln in die Kapillare abgefüllt und anschließend eingeschweißt werden. Das ist zeitaufwändig.

Aus der DE 33 36 738 A1 ist eine Titerplatte nach dem Oberbegriff des Anspruchs 14 bekannt. Der Deckel der bekannten Titerplatte lässt sich nur mit erheblichem Kraftaufwand vom Träger entfernen. Eine in der bekannten Vorrichtung aufgenommene Probe ist schwer von außen zu beheizen; die Vorrichtung ist zur Durchführung der PCR ungeeignet.

Die WO 96/02836 A offenbart ein Verfahren zur Probenvorbereitung mittels Polymerase-Kettenreaktion, bei der die Probe mit der Nachweislösung in Kontakt gebracht wird.

Die FR-A-2 672 301 beschreibt die Amplifikation und den Nachweis einer Nukleinsäure aus einer biologischen Probe. Die biologische Probe und die erforderlichen Reagenzien befinden sich während der gesamten Reaktion in einem hermetisch abgeschlossenen Raum. Die amplifizierte Nukleinsäure wird durch Hybridisierung mit einer immobilisierten Sonde nachgewiesen.

Die Hybridisierung erfolgt erst nachdem sich die Nukleinsäure durch Diffusion zufällig zur Sonde bewegt hat.

Die US-A-5,585,242 offenbart ein Verfahren zum Nachweis amplifizierter Nukleinsäure mittels innerer Totalreflexion. Die Bewegung der amplifizierten Nukleinsäure zu ihrem Bindungspartner erfolgt hier ausschließlich durch Diffusion.

In der US-A-5,604,130 wird ein flüssigkeitsdichter Verschluss für einen Multiwell-Behälter, beispielsweise eine Mikrotiterplatte, beschrieben.

Aufgabe der vorliegenden Erfindung ist es, die Nachteile nach dem Stand der Technik zu beseitigen. Insbesondere sollen ein Verfahren und eine Vorrichtung angegeben werden, mit denen bei der PCR der Zeitaufwand zur Probenvorbereitung verringert wird. Weiters Ziel der Erfindung ist eine vereinfachte und verbesserte, insbesondere in Echtzeit erfolgende, Detektion sowie eine Steigerung der Sensitivität.

Diese Aufgabe wird durch die Merkmale der Ansprüche 1 und 14 gelöst. Zweckmäßige Ausgestaltungen ergeben sich aus den Merkmalen der Ansprüche 2 bis 13 und 15 bis 39.

Nach Maßgabe der Erfindung ist ein Verfahren zur Probenvorbereitung für den Nachweis einer Nukleotidsequenz mittels Polymerase-Ketten-Reaktion vorgesehen, wobei
a) eine Nachweislösung in mindestens eine an einem Träger vorgesehene Kavität gefüllt wird,
b) ein zur Form der Kavität komplementär ausgebildeter Dekkel so auf den Träger gesetzt wird, dass die Nachweislösung zumindest teilweise in einen zwischen der Kavität und dem Deckel gebildeten Spalt verdrängt wird, wobei ein dritter Primer an die in die Kavität ragende Innenseite des Deckels gebunden ist,
c) der Spalt durch mindestens eine in der Nähe einer Öffnung der Kavität vorgesehene Dichtung abgedichtet wird und
d) die Nukleotidsequenz unter dem Einfluss eines elektrischen Felds in Richtung des dritten Primers bewegt und am dritten Primer angelagert wird.

Mit dem vorgeschlagenen Verfahren wird die Zeit zur Probenvorbereitung erheblich verkürzt. Der Vorgang des Einschweißens der Probenlösung in eine Kapillare entfällt.

Der Nachweislösung kann ein erster und/oder zweiter Primer zugesetzt sein. Es wird als besonders vorteilhaft angesehen, dass der dritte Primer mit seinen 5'-terminalen Ende an die in die Kavität ragende Innenseite des Deckels gebunden ist. Auf diese Weise ist es möglich, eine ggf. in einer Probe enthaltene Nukleotidsequenz an den dritten Primer anzulagern. Das kann besonders einfach durch Eintauchen der Innenseite des Deckels in die Probe erreicht werden. Ferner kann die amplifizierte Nukleinsäure nach Abschluss der Amplifikationszyklen durch Binden an den dritten Primer an der Innenseite angereichert werden. Die Anreicherung wird zweckmäßigerweise durch Anlegen eines elektrischen Felds durchgeführt. Unter dem Einfluss eines elektrischen Felds wird die Nukleotidsequenz in Richtung des dritten Primers bewegt.

Zum Nachweis des Vorliegens der gesuchten Nukleotidsequenz wird zweckmäßigerweise die Nachweislösung und/oder einer der Primer auf deren Fluoreszenzeigenschaften untersucht. Bei Anlagerung der nachzuweisenden Nukleotidsequenz an einen der Primer kann eine Veränderung der fluorogenen Eigenschaften der in der Nachweislösung befindlichen Stoffe erfolgen. Vorteilhafterweise ist bei Anlagerung der nachzuweisenden Nukleotidsequenz an einen der Primer eine räumliche Beziehung zwischen zwei fluorophoren Gruppen so änderbar, dass eine Fluoreszenzreaktion erzeug-, änder- oder aufhebbar ist.

Nach einer weiteren Ausgestaltung wird die Nachweislösung zyklisch aufgeheizt und abgekühlt. Ein typischer Temperaturzyklus besteht aus einem ersten Aufheizen der Nachweislösung auf 90 bis 92°C, einem Abkühlen auf 50 bis 55°C sowie einem zweiten Aufheizen auf 72 bis 75°C. Während des ersten Aufheizens erfolgt eine Denaturierung, während des Abkühlens die Renaturierung und während des zweiten Aufheizens die Synthese der Nukleotidsequenz. Der vorgenannte Zyklus wird etwa 30 mal wiederholt.

Das Aufheizen kann mittels Licht, vorzugsweise Infrarotstrahlung, einer Widerstandsheizung oder durch Umspülen der Kavität mit einem Gas oder einer Flüssigkeit durchgeführt werden. Ein schnelles Abkühlen wird zweckmäßigerweise durch Umspülen der Kavität mit einem Gas, z.B. Luft, oder einer Flüssigkeit oder mittels eines Peltierelements durchgeführt.

Erfindungsgemäß ist ferner eine Vorrichtung zur Durchführung des vorgenannten Verfahrens vorgesehen, bei der die Kavität eine konisch zur Öffnung sich erweitende umlaufende Seitenwand und der Spalt eine Breite von höchstens 1 mm aufweist, wobei im Deckel eine Elektrode eingegossen ist und an der der Kavität zugewandten Innenseite des Deckels ein dritter Primer gebunden ist.

Die vorgeschlagene Vorrichtung erlaubt eine zeitsparende Probenvorbereitung. Mit ihr kann der Zeitaufwand zur Durchführung einer PCR erheblich vermindert werden. Der Deckel kann nach der Durchführung der PCR ohne großen Kraftaufwand abgehoben werden.

Vorteilhafterweise ist eine Einrichtung zum Untersuchen der Fluoreszenzeigenschaften der Nachweislösung und/oder einer der Primer vorgesehen sein.

Der Träger kann aus einem lichtdurchlässigen Material, vorzugsweise aus Glas oder Kunststoff, hergestellt sein. Die Kavität ist zweckmäßigerweise abschnittsweise planar ausgebildet; sie weist vorzugsweise einen ebenen Boden auf. Auf dem Träger und/oder an der Innenseite des Deckels kann an zwischen den Kavitäten bzw. an der Innenseite des Deckels angeordneten Vorsprüngen befindlichen Abschnitten eine weitere Dichtung vorgesehen sein. Die weitere Dichtung kann ebenso wie die Dichtung z.B. aus Gummi, Silikon, Teflon oder anderen geeigneten Materialien hergestellt sein.

Es wird als besonders vorteilhaft angesehen, dass der Träger 96 Kavitäten und der Deckel 96 zur Form der Kavitäten komplementäre Vorsprünge aufweist. So kann der Träger beispielsweise in etwa die Dimension einer herkömmlichen 96-Napf Mikrotiterplatte aufweisen. Selbstverständlich kann der Träger auch Bruchteile oder ein Vielfaches der vorgenannten Kavitätenanzahl aufweisen.

Nach einer weiteren Ausgestaltung kann der Deckel aus einem elektrisch leitfähigen Material, vorzugsweise aus einem Kunststoff, hergestellt sein. Der Träger kann eine, vorzugsweise aus Platin hergestellte, Elektrode aufweisen, so dass zwischen dem Deckel und dem Träger ein elektrisches Feld angelegt werden kann, durch das in der Nachweislösung enthaltene Nukleotidsequenz auf die Innenseite bewegt und durch Feldinversionzyklen angereichert werden können.

Der Kunststoff kann ein Polycarbonat, ein Trimenthylthiophen, Triaminobenzol und/oder ein Polycarben enthalten und die Innenseite des Deckels kann zumindest abschnittsweise mit einer biomolekülbindenden Substanz versehen sein. Dabei kann die Bindung der Nukleotidsequenz zum Kunststoff durch Streptavidin oder Avidin vermittelt werden.

Der Nachweislösung ist vorteilhafterweise ein erster und/oder zweiter Primer zugesetzt. Als besonders vorteilhaft wird angesehen, dass an der der Kavität zugewandten Innenseite des Deckels ein dritter Primer, vorzugsweise mit einem 5'terminalen Ende, gebunden ist. Das ermöglicht eine Entfernung der amplifizierten Nukleotidsequenz aus der Nachweislösung.

Nach einem weiteren Ausgestaltungsmerkmal ist ein zum Anregen von Fluoreszenz zwischen dem Boden und der Innenseite des Deckels dienendes Mittel vorgesehen. Vom Mittel zum Anregen stammende Strahlung kann auf die Innenseite des Deckels fokussierbar sein. Das ist insbesondere dann von Vorteil, wenn die Nukleotidsequenz über den dritten Primer an die Innenseite gebunden ist. Das Mittel zum Anregen von Fluoreszenz wird zweckmäßigerweise von einer Laserdiode erzeugt. Es handelt sich in diesem Fall also um Laserlicht. Eine Anregung der Böden des Träger kann auch durch einen so genannten "gallymode" Laser (Science 1998, 280, p 1501, 1544 ff.) in einer vorgegebenen Weise gleichzeitig oder sukzessive erreicht werden.

Ferner können eine Einrichtung zur Detektion der Fluoreszenz, eine Einrichtung zur Auswertung der beobachteten Fluoreszenz und eine Einrichtung zum Bewegen des Trägers relativ zum Mittel zum Anregen der Fluoreszenz und/oder zur Einrichtung zur Detektion vorgesehen sein. Außerdem kann ein facettenaugenartiges Mittel zu separaten Anregung und/oder Detektion der Fluoreszenz zwischen jedem Boden und der Innenseite des dazugehörigen Deckels vorgesehen sein. Dadurch wird weiter Analysenzeit eingespart.

Der Deckel und/oder der Träger sind zweckmäßigerweise zumindest abschnittsweise schwarz gefärbt, so dass darauf abgestrahlte Wärme wirkungsvoll absorbiert wird. Sie bestehen insbesondere aus einem hoch wärmeleitfähigen Material.

Ferner kann eine Einrichtung zum zyklischen Aufheizen und Abkühlen der Nachweislösung vorgesehen sein, wobei zum Beheizen vorteilhafterweise ein Mittel zur Erzeugung von Licht, vorzugsweise Infrarotstrahlung, eine Widerstandsheizung oder ein Mittel zum Umspülen der Kavität mit einem Gas oder einer Flüssigkeit vorgesehen ist. Ferner ist zweckmäßigerweise ein Mittel zum Abkühlen vorgesehen, wobei die Abkühlung vorzugsweise durch Umspülen der Kavität mit einem Gas oder einer Flüssigkeit oder mittels eines Peltierelements erzielt wird.

Zur Verbesserung der Wärmeleitfähigkeit bei gleichzeitig guter Transparenz kann der Träger einen aus einem Glas hergestellten Boden aufweisen. Die Dichtung ist zweckmäßigerweise durch eine an der, vorzugsweise aus Kunststoff hergestellten, Seitenwand umlaufende Ausnehmung und einen dazu komplementären am Vorsprung vorgesehenen umlaufenden formschlüssig in die Ausnehmung einrastbaren Wulst gebildet. Die Dichtung ist zweckmäßigerweise bei einem Druckanstieg in der Kavität, z.B. durch Temperaturerhöhung, selbstabdichtend, indem der Wulst gegen die Ausnehmung gedrückt wird. Das Aufbringen oder Abheben des Deckels lässt sich durch eine hohe Flexibilität der zwischen den Vorsprüngen liegenden Abschnitte durch leichtes Biegen besonders einfach bewerkstelligen.

Schließlich wird ein Kit zur Durchführung des erfindungsgemäßen Verfahrens beansprucht, umfassend eine Vorrichtung nach Anspruch 14 und eine mindestens einen ersten Primer enthaltenden Nachweislösung.

Die Nachweislösung kann einen zweiten Primer enthalten.

Nachfolgend werden Ausführungsbeispiele der Erfindung anhand der Zeichnung näher erläutert. Es zeigen:
- Fig. 1: eine schematische Querschnittsansicht durch eine erste Vorrichtung,
- Fig. 2: die Vorrichtung gemäß Fig. 1 bei Anregung und Detektion,
- Fig. 3: eine schematische Querschnittsansicht einer zweiten Vorrichtung bei Anregung und Detektion,
- Fig. 3a: eine schematische Querschnittsansicht der Vorrichtung gemäß Fig. 3,
- Fig. 4: eine schematische Teilquerschnittsansicht einer dritten Vorrichtung,
- Fig. 5: eine schematische Querschnittsansicht einer Dichtung und
- Fig. 6: die Querschnittsansicht nach Fig. 5 im nicht eingerasteten Zustand.

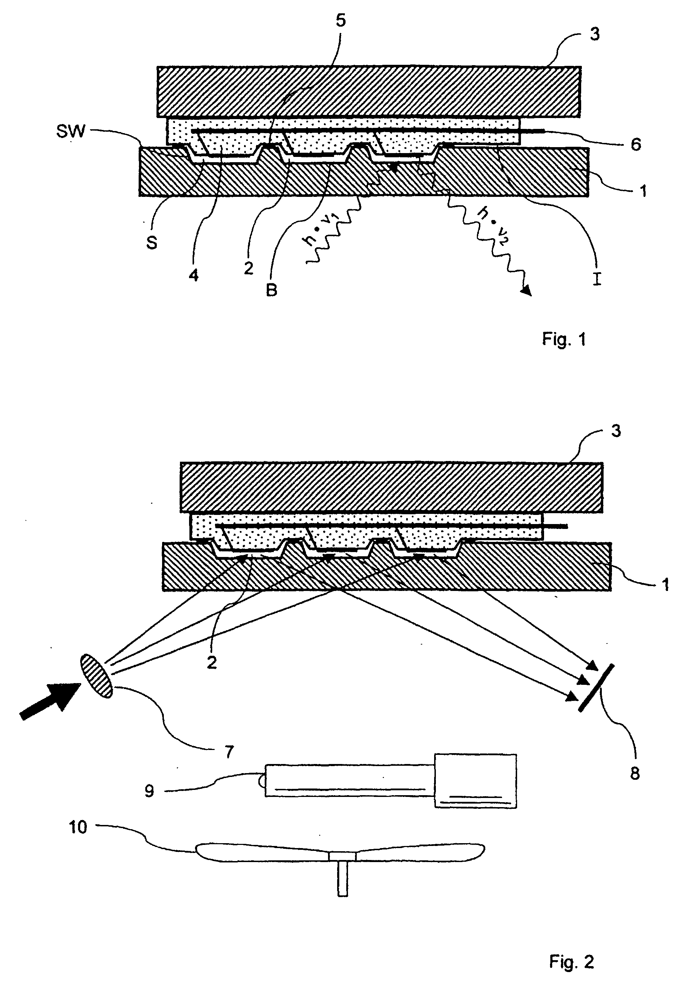

In Fig. 1 weist ein Träger 1 mehrere Kavitäten 2 auf. Ein Deckel 3 ist an seiner Innenseite I mit mehreren Vorsprüngen 4 versehen. Die Vorsprünge 4 weisen eine zur Kavität 2 komplementäre Form auf. Zwischen einem Boden B der Kavität 2 und dem parallel verlaufenden gegenüberliegenden Vorsprung 4 ist ein Spalt S gebildet. Die Kavität 2 ist des Weiteren durch eine sich vom Boden B zur Öffnung der Kavität 2 hin konisch öffnende umlaufende Seitenwand SW begrenzt. Der zwischen dem Vorsprung 4 und der Kavität 2 gebildete Spalt S weist eine Breite von höchstens 1 mm auf. In der Nähe der Öffnung der Kavität 2 ist eine Dichtung 5 vorgesehen. Mit 6 ist eine im Deckel 3 eingegossene Elektrode bezeichnet. Der Träger 1 kann eine (hier nicht dargestellte) Gegenelektrode aufweisen.

In Fig. 2 ist die Vorrichtung nach Fig. 1 bei der Anregung und Detektion gezeigt. Mit 7 ist schematisch eine optische Einrichtung zum Anregen der im Spalt S aufgenommenen Nachweislösung bezeichnet. Dabei handelt es sich um ein facettenaugenartiges Mittel, mit dem mehrere oder alle Kavitäten 2 gleichzeitig z.B. mit Laserlicht beaufschlagt werden können. Die optische Einrichtung 7 ist auf die dem Boden B gegenüberliegende Innenseite I des Deckels 3 fokussiert. Mit 8 ist ein Fluorometer bezeichnet. Auch das Fluorometer 8 kann mit einem facettenaugenartigen Mittel versehen sein, so dass die von mehreren oder allen Kavitäten 2 ausgehende Fluoreszenz nachweisbar ist. Gegenüber dem Träger 1 befinden sich eine Infrarotstrahlungsquelle 9 sowie ein Ventilator 10.

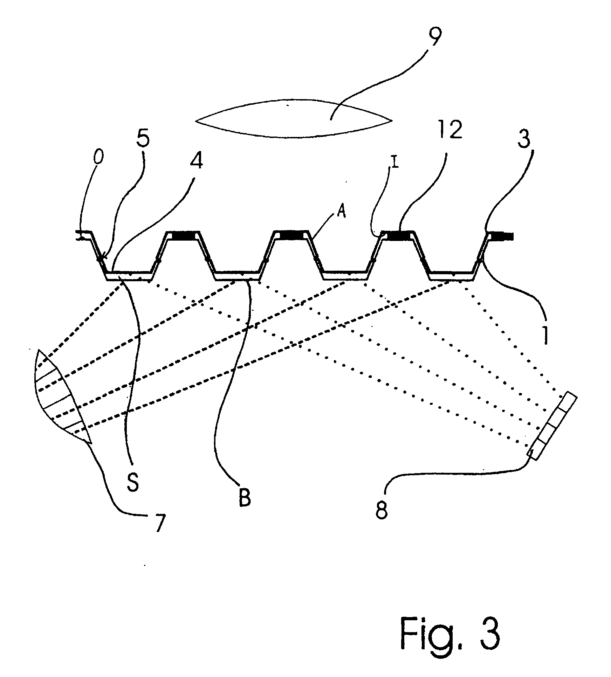

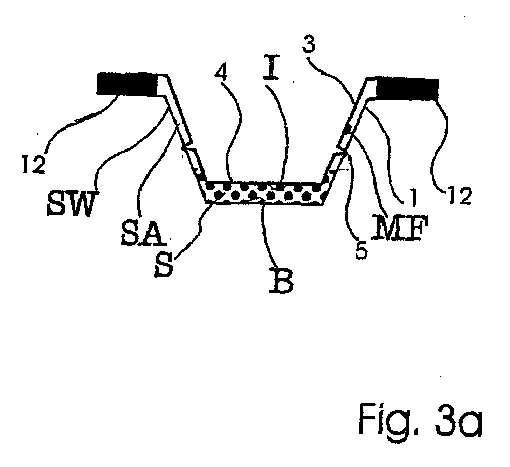

Die Fig. 3 und 3a zeigen eine zweite Vorrichtung im schematischen Querschnitt. Dabei ist die Infrarotstrahlungsquelle 9 gegenüber dem Deckel 3 angeordnet. Der Deckel 3 ist aus einem schwarzen Material hoher Wärmeleitfähigkeit, z.B. einem Glas oder Metall, hergestellt. Zur besseren Absorption ist der Deckel 3 auf der der Innenseite I gegenüberliegenden Außenseite A mit einer schwarzen Farbe beschichtet. Die erste Dichtung 5 ist als selbstabdichtende Rastverbindung ausgebildet. Eine zweite Dichtung 12 in der Nähe des Öffnungsrands der Kavität 2 besteht aus Gummi oder elastischem Kunststoff. Sie ist zwischen der Innenseite I des Deckels 3 und einer Oberseite O des Trägers 1 vorgesehen. Eine in der Kavität 2 aufgenommene Nachweislösung ist bei geschlossenem Deckel 3 in einen zwischen der Seitenwand SW und einer gegenüberliegenden Mantelfläche MF des Vorsprungs 4 befindlichen Spaltabschnitt SA verdrängt.

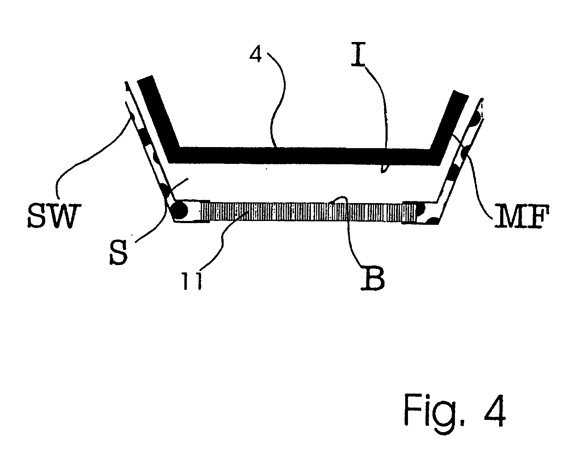

Auch in Fig. 4 ist der Träger 1 komplementär zum Deckel 3 geformt. Er ist ebenfalls aus einem Material hoher Wärmeleitfähigkeit hergestellt. Der Boden B ist aus einem transparenten Material, z.B. einem Glasfenster 11, gebildet.

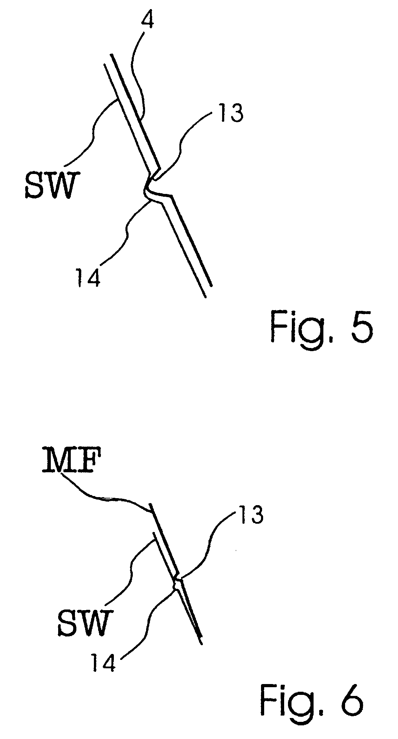

In Fig. 5 ist in einer schematischen Teilquerschnittsansicht durch die erste Dichtung 5 gezeigt. Dabei greift ein an der Mantelfläche MF des Vorsprungs 4 vorgesehener umlaufender Wulst 13 im geschlossenen Zustand form- und kraftschlüssig in eine an der Seitenwand SW vorgesehene umlaufende Ausnehmung 14 ein. Fig. 6 zeigt die erste Dichtung 5 im noch nicht formschlüssig eingerasteten Zustand.

Die Funktion der Vorrichtung ist folgende:

Zur Probenvorbereitung wird Nachweislösung in die Kavität 2 des Trägers 1 pipettiert. Der Pipettiervorgang kann beispielsweise mittels eines Pipettierroboters erfolgen. Bei der Nachweislösung handelt es sich vorzugsweise um einen so genannten Mastermix, in dem alle erforderlichen Reagenzien zur Durchführung der PCR enthalten sind. Insbesondere sind ein erster und ein zweiter Primer in der Nachweislösung enthalten. An der Innenseite I des Deckels 3 ist im Bereich der Vorsprünge 4 ein dritter Primer gebunden. An den dritten Primer ist die nachzuweisende Nukleotidsequenz angelagert. Die Anlagerung kann z.B. dadurch erfolgen, dass der Deckel 3 zuvor in eine die nachzuweisende Nukleotidsequenz enthaltende Probenlösung getaucht wird.

Zur Probenvorbereitung muß nun der Deckel 3 lediglich auf den Träger 1 so aufgesetzt werden, daß die Vorsprünge 4 in die komplementären Kavitäten 2 eintauchen. Dabei kommt der dritte Primer mit der angelagerten Nukleotidsequenz in Kontakt mit der Nachweislösung. Der Deckel 3 wird mit dem Träger 1 dichtend verschlossen, z.B. indem die an den Vorsprüngen 4 vorgesehenen Wülste 13 in die komplementären Ausnehmungen 14 der Kavitäten 2 einrasten. In diesem Zustand wird ein Teil der Nachweislösung in den Spalt S verdrängt.

Anschließend wird insbesondere der zwischen der Innenseite I und dem Boden B gebildeter Spalt S der zur Durchführung der PCR erforderlichen Temperaturbehandlung unterzogen. Danach oder auch zwischen jedem Temperaturzyklus wird der zwischen Innenseite I und Boden B befindliche Bereich jeder Kavität 2 mittels der optischen Einrichtung 7 angeregt und dann unter Verwendung des Fluorometers 8 auf Fluoreszenz untersucht. Eine Fluoreszenzänderung zeigt das Vorhandensein bzw. Nichtvorhandensein der gesuchten Nukleotidsequenz an.

Die Temperaturzyklen werden durch wiederkehrendes Aktivieren bzw. Deaktivieren der IR-Strahlungsquelle 9 bzw. des Ventilators 10 hervorgerufen.

### Bezugszeichenliste

- 1: Träger
- 2: Kavität
- 3: Deckel
- 4: Vorsprung
- 5: erste Dichtung
- 6: Elektrode
- 7: optische Einrichtung
- 8: Fluorometer
- 9: IR-Strahlungsquelle
- 10: Ventilator
- 11: Glasfenster
- 12: zweite Dichtung
- 13: Wulst
- 14: Ausnehmung

- I: Innenseite
- B: Boden
- SW: Seitenwand
- S: Spalt
- A: Außenseite
- O: Oberseite
- MF: Mantelfläche
- SA: Spaltabschnitt

## Patentansprüche

1. Verfahren zur Probenvorbereitung für den Nachweis einer Nukleotidsequenz mittels Polymerase-Ketten-Reaktion (PCR), bei der die Probe mit der Nachweislösung in Kontakt gebracht wird, wobei
a) eine Nachweislösung in mindestens eine an einem Träger (1) vorgesehene Kavität (2) gefüllt wird,
b) ein zur Form der Kavität (2) komplementär ausgebildeter Deckel (3) so auf den Träger (1) gesetzt wird, dass die Nachweislösung zumindest teilweise in einen zwischen der Kavität (2) und dem Deckel (3) gebildeten Spalt (S) verdrängt wird, wobei ein dritter Primer an die in die Kavität (2) ragende Innenseite (I) des Deckels (3) gebunden ist,
c) der Spalt (S) durch mindestens eine in der Nähe einer Öffnung der Kavität (2) vorgesehene Dichtung (5, 12) abgedichtet wird und
d) die Nukleotidsequenz unter dem Einfluss eines elektrischen Felds in Richtung des dritten Primers bewegt und am dritten Primer angelagert wird.

2. Verfahren nach Anspruch 1, wobei der Nachweislösung ein erster Primer zugesetzt wird.

3. Verfahren nach einem der vorhergehenden Ansprüche, wobei der Nachweislösung ein zweiter Primer zugesetzt wird.

4. Verfahren nach einem der vorhergehenden Ansprüche, wobei der dritte Primer mit seinem 5'-terminalen Ende an die in die Kavität (2) ragende Innenseite (I) des Deckels (3) gebunden ist.

5. Verfahren nach Anspruch 4, wobei die in der Probe ggf. enthaltene Nukleotidsequenz an den dritten Primer angelagert wird.

6. Verfahren nach einem der Ansprüche 4 oder 5, wobei die amplifizierte Nukleinsäure nach Abschluss der Amplifikationszyklen durch Binden an den dritten Primer an der Innenseite (I) angereichert wird.

7. Verfahren nach Anspruch 6, wobei die Anreicherung durch Anlegen eines elektrischen Felds durchgeführt wird.

8. Verfahren nach einem der vorhergehenden Ansprüche, wobei die Nachweislösung und/oder einer der Primer auf deren Fluoreszenzeigenschaften untersucht wird/werden.

9. Verfahren nach einem der vorhergehenden Ansprüche, wobei bei Anlagerung der nachzuweisenden Nukleotidsequenz an einen der Primer eine Veränderung der fluorogenen Eigenschaften der in der Nachweislösung befindlichen Stoffe erfolgt.

10. Verfahren nach einem der vorhergehenden Ansprüche, wobei bei Anlagerung der nachzuweisenden Nukleotidsequenz an einen der Primer eine räumliche Beziehung zwischen zwei fluorophoren Gruppen so geändert wird, dass eine Fluoreszenzreaktion erzeug-, änder- oder aufhebbar ist.

11. Verfahren nach einem der vorhergehenden Ansprüche, wobei die Nachweislösung zyklisch aufgeheizt und abgekühlt wird.

12. Verfahren nach Anspruch 11, wobei das Aufheizen mittels Licht, vorzugsweise Infrarotstrahlung, einer Widerstandsheizung oder durch Umspülen der Kavität (2) mit einem Gas oder einer Flüssigkeit durchgeführt wird.

13. Verfahren nach Anspruch 11 oder 12, wobei das Abkühlen durch Umspülen der Kavität mit einem Gas oder einer Flüssigkeit oder mittels eines Peltierelements durchgeführt wird.

14. Vorrichtung zur Durchführung eines Verfahrens nach einem der Ansprüche 1 bis 13, mit einem Träger (1) mit mindestens einer Kavität (2), einem zur Kavität (2) komplementär ausgebildeten Deckel (3), der so auf den Träger (1) aufsetzbar ist, dass eine in der Kavität (2) aufgenommene Nachweislösung zumindest teilweise in einen zwischen der Kavität (2) und dem Deckel (3) gebildeten Spalt (S) verdrängbar ist und einer in der Nähe einer Öffnung der Kavität (2) angeordneten Dichtung (5, 12) zum Abdichten des Spalts (S), **dadurch gekennzeichnet, dass** die Kavität (2) eine konisch zur Öffnung sich erweiternde umlaufende Seitenwand (SW) und der Spalt (S) eine Breite (B) von höchstens 1 mm aufweist, wobei im Deckel (3) eine Elektrode (6) eingegossen ist und an der der Kavität (2) zugewandten Innenseite (I) des Deckels (3) ein dritter Primer gebunden ist.

15. Vorrichtung nach Anspruch 14, wobei eine Einrichtung zum Untersuchen der Fluoreszenzeigenschaften der Nachweislösung und/oder einer der Primer vorgesehen ist.

16. Vorrichtung nach einem der Ansprüche 14 oder 15, wobei der Träger aus einem lichtdurchlässigen Material, vorzugsweise aus Glas oder Kunststoff, hergestellt ist.

17. Vorrichtung nach einem der Ansprüche 14 bis 16, wobei die Kavität (2) abschnittsweise planar ausgebildet ist, vorzugsweise einen ebenen Boden (B), aufweist.

18. Vorrichtung nach einem der Ansprüche 14 bis 17, wobei auf dem Träger (1) und/oder an der Innenseite (I) des Deckels (3) in zwischen den Kavitäten (2) bzw. an der Innenseite (I) des Deckels (3) vorgesehenen Vorsprüngen (4) befindlichen Abschnitten eine weitere Dichtung (12) vorgesehen ist.

19. Vorrichtung nach einem der Ansprüche 14 bis 18, wobei der Träger 96 Kavitäten (2) und der Deckel (3) 96 zur Form der Kavitäten (2) komplementäre Vorsprünge (4) aufweisen.

20. Vorrichtung nach einem der Ansprüche 14 bis 19, wobei der Deckel (3) aus einem elektrisch leitfähigen Material, vorzugsweise einem Kunststoff, hergestellt ist.

21. Vorrichtung nach Anspruch 20, wobei der Kunststoff ein Polycarbonat, Trimethylthiophen, Triaminobenzol und/oder ein Polycarben enthält und die Innenseite (I) des Deckels (3) zumindest abschnittsweise mit einer biomolekülbindenden Substanz versehen ist.

22. Vorrichtung nach einem der Ansprüche 14 bis 21, wobei der Träger (1) eine, vorzugsweise aus Platin hergestellte, Elektrode aufweist, so dass ein elektrisches Feld angelegt werden kann, durch das in der Nachweislösung enthaltene Nukleotidsequenzen auf die Innenseite (I) bewegt und durch Feldinversionszyklen angereichert werden können.

23. Vorrichtung nach einem der Ansprüche 14 bis 22, wobei der Nachweislösung ein erster Primer zugesetzt ist.

24. Vorrichtung nach einem der Ansprüche 14 bis 23, wobei der Machweislösung ein zweiter Primer zugesetzt ist.

25. Vorrichtung nach einem der Ansprüche 14 bis 24, wobei an der der Kavität (2) zugewandten Innenseite (I) des Deckels der dritte Primer (3) mit seinem 5'-terminalen Ende gebunden ist.

26. Vorrichtung nach einem der Ansprüche 14 bis 25, wobei ein zum Anregen von Fluoreszenz zwischen Boden (B) und der Innenseite (I) des Deckels (3) dienendes Mittel vorgesehen ist.

27. Vorrichtung nach Anspruch 26, wobei vom Mittel zum Anregen stammende Strahlung auf die Innenseite (I) des Deckels (3) fokussierbar ist.

28. Vorrichtung nach einem der Ansprüche 26 oder 27, wobei das Mittel (7) zum Anregen von Fluoreszenz von einer Laserdiode erzeugt wird.

29. Vorrichtung nach einem der Ansprüche 14 bis 28, wobei eine Einrichtung (8) zur Detektion der Fluoreszenz vorgesehen ist.

30. Vorrichtung nach einem der Ansprüche 14 bis 29, wobei eine Einrichtung zur Auswertung der beobachteten Fluoreszenz vorgesehen ist.

31. Vorrichtung nach einem der Ansprüche 14 bis 30, wobei eine Einrichtung zum Bewegen des Trägers (1) relativ zum Mittel (7) zum Anregen von Fluoreszenz und/oder zur Einrichtung (8) zur Detektion vorgesehen ist.

32. Vorrichtung nach einem der Ansprüche 14 bis 31, wobei ein facettenaugenartiges Mittel zur separaten Anregung und/oder Detektion der Fluoreszenz zwischen jedem Boden (B) und der Innenseite (I) des dazugehörigen Deckels (3) vorgesehen ist.

33. Vorrichtung nach einem der Ansprüche 14 bis 32, wobei der Deckel (3) und/oder der Träger (1) zumindest abschnittsweise schwarz gefärbt ist, so dass darauf abgestrahlte Wärme wirkungsvoll absorbiert wird.

34. Vorrichtung nach einem der Ansprüche 14 bis 33, wobei eine Einrichtung zum zyklischen Aufheizen und Abkühlen der Nachweislösung vorgesehen ist.

35. Vorrichtung nach Anspruch 34, wobei zum Beheizen ein Mittel (9) zur Erzeugung von Licht, vorzugsweise Infrarotstrahlung, eine Widerstandsheizung oder ein Mittel zum Umspülen der Kavität (2) mit einem Gas oder einer Flüssigkeit vorgesehen ist.

36. Vorrichtung nach Anspruch 34 oder 35, wobei ein Mittel (10) zum Abkühlen, vorzugsweise durch Umspülen der Kavität (2) mit einem Gas oder einer Flüssigkeit oder mittels eines Peltierelements, vorgesehen ist.

37. Vorrichtung nach einem der Ansprüche 14 bis 36, wobei der Träger (1) einen aus Glas hergestellten Boden (B) aufweist.

38. Vorrichtung nach einem der Ansprüche 14 bis 37, wobei die Dichtung (5) durch eine an der Seitenwand (SW) umlaufende Ausnehmung (14) und einen dazu komplementären am Vorsprung (4) vorgesehenen umlaufenden formschlüssig in die Ausnehmung (14) einrastbaren Wulst (12) gebildet ist.

39. Vorrichtung nach Anspruch 38, wobei die Dichtung (5) bei einem Druckanstieg in der Kavität (2) selbstabdichtend ist, indem der Wulst (13) gegen die Ausnehmung (14) gedrückt wird.

40. Kit zur Durchführung des Verfahrens nach Anspruch 1, umfassend eine Vorrichtung nach Anspruch 14 und eine mindestens einen ersten Primer enthaltende Nachweislösung.

41. Kit nach Anspruch 40, wobei die Nachweislösung einen zweiten Primer enthält.

## Claims

1. Method for preparing samples for detecting a nucleotide sequence by means of polymerase chain reaction (PCR) where the sample is contacted with the analysis solution, wherein
a) an analysis solution is filled into at least one cavity (2) provided on a support (1),
b) a lid (3) designed complementary to the shape of the cavity (2) is placed onto the support (1) in such a way that at least some of the analysis solution is displaced into a gap (G) formed between the cavity (2) and the lid (3), wherein a third primer is bound to the internal face (I) of the lid (3) extending into the cavity (2),
c) the gap (G) is sealed by means of at least one seal (5, 12) provided near an opening of the cavity (2) and
d) the nucleotide sequence is moved toward the third primer under the influence of an electric field and is bound to the third primer.

2. Method according to claim 1, wherein the analysis solution has a first primer added to it.

3. Method according to one of the preceding claims, wherein the analysis solution has a second primer added to it.

4. Method according to any of the preceding claims, wherein the third primer is bound to the internal face (I) of the lid (3) extending into the cavity (2) with its 5'-terminal end.

5. Method according to claim 4, wherein the nucleotide sequence which may be present in the sample is bound to the third primer.

6. Method according to any of claims 4 or 5, wherein, after the amplification cycles have been concluded, amplified nucleic acid is accumulated on the internal face (I) by binding to the third primer.

7. Method according to claim 6, wherein accumulation is carried out by applying an electrical field.

8. Method according to any of the preceding claims, wherein the analysis solution and/or one of the primers is/are examined for their fluorescence properties.

9. Method according to any of the preceding claims, wherein, upon binding of the nucleotide sequence to be detected to one of the primers, a change in the fluorogenic properties of the substances present in the analysis solution takes place.

10. Method according to any of the preceding claims,. wherein, upon binding of the nucleotide sequence to be detected to one of the primers, a spatial relationship between two fluorophoric groups is altered in such a way that a fluorescence reaction can be generated, altered or quenched.

11. Method according to any of the preceding claims, wherein the analysis solution is heated and cooled cyclically.

12. Method according to claim 11, wherein heating is effected by means of light, preferably infra-red radiation, a resistance heating or by passing a gas or a fluid around the cavity (2).

13. Method according to claim 11 or 12, wherein cooling is effected by passing a gas or a fluid around the cavity or by means of a Peltier element.

14. Device for detecting a nucleotide sequence by a method according to any of the claims 1 to 13, with a support (1) with at least one cavity (2), a lid (3) which is designed complementary to the cavity (2) and which can be placed on the support (1) in such a way that at least some of the analysis solution taken up by the cavity (2) can be displaced into a gap (2) formed between the cavity (2) and the lid (3) and with a seal (5, 12), arranged near an opening of the cavity (2), for sealing the gap (G), **characterised in that** the cavity has a surrounding lateral wall (LW) which widens conically toward the opening and the gap (G) has a width (W) of not more than 1 mm, wherein an electrode (6) is moulded integrally into the lid (3) and a third primer is bound to the internal face (I) of the lid (3) facing the cavity (2).

15. Device according to claim 14, wherein a facility for examining the fluorescence properties of the analysis solution and/or one of the primers is provided.

16. Device according to any of claims 14 or 15, wherein the support is made of a translucent material, preferably of glass or plastic.

17. Device according to one of claims 14 to 16, wherein the cavity is designed to have planar sections, preferably has a flat bottom (B).

18. Device according to any of claims 14 to 17, wherein a further seal (12) is provided on the support (1) and/or on the internal face (I) of the lid (3) in sections located between the cavities (2) or on projections (4) provided on the internal face (I) of the lid (3).

19. Device according to any of claims 14 to 18, wherein the support has 96 cavities (2) and the lid (3) 96 projections (4) which are complementary to the shape of the cavities (2).

20. Device according to any of claims 14 to 19, wherein the lid (3) is made of an electroconductive material, preferably a plastic.

21. Device according to claim 20, wherein the plastic comprises a polycarbonate, trimethylthiophene, triaminobenzene and/or a polycarbene and at least sections of the internal face (I) of the lid (3) are provided with a substance which binds biomolecules.

22. Device according to any of claims 14 to 21, wherein the support (1) has an electrode, preferably an electrode made of platinum, so that an electrical field can be applied by which nucleotide sequence present in the analysis solution can be shifted to the internal face (I) and accumulated by field-inversion cycles.

23. Device according to any of claims 14 to 22, wherein the analysis solution has a first primer added to it.

24. Device according to any of claims 14 to 23, wherein the analysis solution has a second primer added to it.

25. Device according to any of the preceding claims 14 to 24, wherein a third primer is bound to the internal face (I) of the lid (3) facing the cavity (2) with its 5'-terminal end.

26. Device according to any of claims 14 to 25, wherein a means for exciting fluorescence between bottom (B) and the internal face (I) of the lid (3) is provided.

27. Device according to claim 26, wherein radiation originating from the excitation means can be focused toward the internal face (I) of the lid (3).

28. Device according to any of claims 26 or 27, wherein the means (7) for exciting fluorescence is generated by a laser diode.

29. Device according to any of claims 14 to 28, wherein a facility (8) for detecting the fluorescence is provided.

30. Device according to any of claims 14 to 29, wherein a facility for evaluating the fluorescence observed is provided.

31. Device according to any of claims 14 to 30, wherein a facility is provided for moving the support (1) relative to the means (7) for exciting the fluorescence and/or to the detection facility (8).

32. Device according to any of claims 14 to 31, wherein a facet-eye-like means for separately exciting and/or detecting the fluorescence between each bottom (B) and the internal face (I) of the corresponding lid (3) is provided.

33. Device according to any of claims 14 to 32, wherein at least sections of the lid (3) and/or the support (1) are black so that heat radiated at them is absorbed in an efficient manner.

34. Device according to any of claims 14 to 33, wherein a facility for cyclically heating and cooling the analysis solution is provided.

35. Device according to claim 34, wherein a means (9) for generating light, preferably infra-red radiation, a resistance heating or a means for passing a gas or a fluid around the cavity (2) is provided for heating.

36. Device according to claim 34 or 35, wherein a means (10) for cooling, preferably by passing a gas or a fluid around the cavity (2), or by means of a Peltier element is provided.

37. Device according to any of claims 14 to 36, wherein the support (1) has a bottom (B) made of glass.

38. Device according to any of claims 14 to 37, wherein the seal (5) is formed by a recess (14) surrounding the lateral wall (LW) and a surrounding reinforcement (13) which is provided at the projection (4) so that it complements the recess (14) and which can lock positively into the recess (14).

39. Device according to claim 38, wherein, when the pressure in the cavity (2) rises, the seal (5) is self-sealing by pressing the reinforcement (13) against the recess (14).

40. Kit for carrying out the process according to claim 1 with device according to claim 14 and an analysis solution comprising at least one first primer.

41. Kit according to claim 40, wherein a second primer is present in the analysis solution.

## Revendications

1. Procédé pour préparer des échantillons destinés à la détection d'une séquence nucléotidique au moyen d'une réaction en chaîne par la polymérase (PCR), dans lequel l'échantillon est mis en contact avec la solution de détection, où
a) une solution de détection est versée dans au moins une cavité (2) prévue dans un support (1),
b) un couvercle (3) conçu de façon à être complémentaire de la forme de la cavité (2) est placé sur le support (1) de façon telle que la solution de détection est repoussée au moins partiellement dans une fente (S) formée entre la cavité (2) et le couvercle (3), où une troisième amorce est liée au côté interne (I) du couvercle (3) faisant saillie dans la cavité (2),
c) la fente (S) est rendue étanche par au moins une garniture (5, 12) prévue à proximité d'une ouverture de la cavité (2), et
d) la séquence nucléotidique, sous l'influence d'un champ électrique, se déplace dans la direction de la troisième amorce et se fixe à la troisième amorce.

2. Procédé selon la revendication 1, dans lequel une première amorce est ajoutée à la solution de détection.

3. Procédé selon l'une des revendications précédentes, dans lequel une seconde amorce est ajoutée à la solution de détection.

4. Procédé selon l'une des revendications précédentes, dans lequel la troisième amorce est liée par son extrémité 5'-terminale au côté interne (I) du couvercle (3) faisant saillie dans la cavité (2).

5. Procédé selon la revendication 4, dans lequel la séquence nucléotidique contenue éventuellement dans l'échantillon se fixe sur la troisième amorce.

6. Procédé selon l'une des revendications 4 ou 5, dans lequel l'acide nucléique amplifié après la fin des cycles d'amplification est enrichi par liaison à la troisième amorce sur le côté interne (I).

7. Procédé selon la revendication 6, dans lequel l'enrichissement est réalisé par application d'un champ électrique.

8. Procédé selon l'une des revendications précédentes, dans lequel on examine les propriétés de fluorescence de la solution de détection et/ou d'une des amorces.

9. Procédé selon l'une des revendications précédentes, dans lequel, lors de la fixation de la séquence nucléotidique à détecter sur une des amorces, il se produit une modification des propriétés fluorogènes des substances se trouvant dans la solution de détection.

10. Procédé selon l'une des revendications précédentes, dans lequel, lors de la fixation de la séquence nucléotidique à détecter sur une des amorces, une relation spatiale entre deux groupes fluorophores est modifiée de façon telle qu'une réaction de fluorescence peut être générée, modifiée ou interrompue.

11. Procédé selon l'une des revendications précédentes, dans lequel la solution de détection est chauffée et refroidie de façon cyclique.

12. Procédé selon la revendication 11, dans lequel le chauffage est réalisé au moyen de lumière, de préférence d'un rayonnement infrarouge, d'un chauffage à résistance, ou par balayage de la cavité (2) avec un gaz ou un liquide.

13. Procédé selon la revendication 11 ou 12, dans lequel le refroidissement est réalisé par balayage de la cavité avec un gaz ou un liquide, ou au moyen d'un élément de Peltier.

14. Dispositif pour réaliser un procédé selon l'une des revendications 1 à 13, comportant un support (1) présentant au moins une cavité (2), un couvercle (3) formé de façon à être complémentaire de la cavité (2), et qui peut être placé sur le support (1) de façon telle qu'une solution de détection placée dans la cavité (2) peut être repoussée au moins partiellement dans une fente (S) formée entre la cavité (2) et le couvercle (3), et une garniture (5, 12) disposée à proximité d'une ouverture de la cavité (2) et destinée à rendre étanche la fente (S), **caractérisé en ce que** la cavité (2) présente une paroi latérale périphérique (SW) s'élargissant de façon conique vers l'ouverture et **en ce que** la fente (S) présente une largeur (B) au plus égale à 1 mm, une électrode (6) étant incorporée dans le couvercle (3) et une troisième amorce étant liée au côté interne (I) du couvercle (3) orienté vers la cavité (2).

15. Dispositif selon la revendication 14, dans lequel est prévu un dispositif permettant d'examiner les propriétés de fluorescence de la solution de détection et/ou d'une des amorces.

16. Dispositif selon l'une des revendications 14 ou 15, dans lequel le support est fabriqué à partir d'un matériau perméable à la lumière, de préférence du verre ou un matériau synthétique.

17. Dispositif selon l'une des revendications 14 à 16, dans lequel la cavité (2) est conçue de façon plane par segments, et présente de préférence un fond plat (B).

18. Dispositif selon l'une des revendications 14 à 17, dans lequel une autre garniture (12) est prévue sur le support (1) et/ou sur le côté interne (I) du couvercle (3), dans des segments qui se trouvent entre les cavités (2) ou au niveau de saillies (4) prévues sur le côté interne (I) du couvercle.

19. Dispositif selon l'une des revendications 14 à 18, dans lequel le support présente 96 cavités (2) et le couvercle (3) présente 96 saillies (4) complémentaires de la forme des cavités (2).

20. Dispositif selon l'une des revendications 14 à 19, dans lequel le couvercle (3) est fabriqué à partir d'un matériau électriquement conducteur,. de préférence un matériau synthétique.

21. Dispositif selon la revendication 20, dans lequel le matériau synthétique contient un polycarbonate, un triméthylthiophène, un triaminobenzène et/ou un polycarbène et le côté interne (I) du couvercle (3) est pourvu au moins par segments d'une substance liant des biomolécules.

22. Dispositif selon l'une des revendications 14 à 21, dans lequel le support (1) présente une électrode, fabriquée de préférence à partir de platine, de façon telle qu'un champ électrique peut être appliqué, par lequel les séquences nucléotidiques contenues dans la solution de détection se déplacent sur le côté interne (I) et peuvent être enrichies par des cycles avec inversion de champ.

23. Dispositif selon l'une des revendications 14 à 22, dans lequel une première amorce est ajoutée à la solution de détection.

24. Dispositif selon l'une des revendications 14 à 23, dans lequel une seconde amorce est ajoutée à la solution de détection.

25. Dispositif selon l'une des revendications 14 à 24, dans lequel la troisième amorce (3) est liée par son extrémité 5'-terminale au côté interne (I) du couvercle orientée vers la cavité (2).

26. Dispositif selon l'une des revendications 14 à 25, dans lequel est prévu un moyen qui sert à activer la fluorescence entre le fond (B) et le côté interne (I) du couvercle (3).

27. Dispositif selon la revendication 26, dans lequel le rayonnement provenant du moyen d'activation peut être focalisé sur le côté interne (I) du couvercle (3).

28. Dispositif selon l'une des revendications 26 ou 27, dans lequel le moyen (7) d'activation de la fluorescence est généré par une diode laser.

29. Dispositif selon l'une des revendications 14 à 28, dans lequel est prévu un dispositif (8) de détection de la fluorescence.

30. Dispositif selon l'une des revendications 14 à 29, dans lequel est prévu un dispositif d'évaluation de la fluorescence observée.

31. Dispositif selon l'une des revendications 14 à 30, dans lequel est prévu un dispositif permettant de déplacer le support (1) par rapport au moyen (7) d'activation de la fluorescence et/ou par rapport au dispositif (8) de détection.

32. Dispositif selon l'une des revendications 14 à 31, dans lequel est prévu un moyen de type oeil à facettes pour une activation et/ou une détection séparée(s) de la fluorescence entre chaque fond (B) et le côté interne (I) du couvercle (3) qui s'y rapporte.

33. Dispositif selon l'une des revendications 14 à 32, dans lequel le couvercle (3) et/ou le support (1) est(sont) coloré(s) en noir au moins par segments, de façon telle que la chaleur dissipée est absorbée efficacement.

34. Dispositif selon l'une des revendications 14 à 33, dans lequel est prévu un dispositif de chauffage et de refroidissement cycliques de la solution de détection.

35. Dispositif selon la revendication 34, dans lequel, pour le chauffage, est prévu un moyen (9) de génération de lumière, de préférence un rayonnement infrarouge, un chauffage à résistance ou un moyen de balayage de la cavité (2) avec un gaz ou un liquide.

36. Dispositif selon la revendication 34 ou 35, dans lequel est prévu un moyen (10) de refroidissement, de préférence par balayage de la cavité (2) avec un gaz ou un liquide, ou au moyen d'un élément de Peltier.

37. Dispositif selon l'une des revendications 14 à 36, dans lequel le support (1) présente un fond (B) fabriqué en verre.

38. Dispositif selon l'une des revendications 14 à 37, dans lequel la garniture (5) est formée par un évidement (14) circulaire au niveau de la paroi latérale (SW) et par un bourrelet (13) périphérique qui lui est complémentaire, prévu au niveau d'une saillie (4), et s'enclenchant par engagement géométrique dans l'évidement (14).

39. Dispositif selon la revendication 38, dans lequel la garniture (5), lors d'une augmentation de pression dans la cavité (2), est auto-étanchéifiante, par le fait que le bourrelet (13) est comprimé contre l'évidement (14).

40. Kit de réalisation du procédé selon la revendication 1, comprenant un dispositif selon la revendication 14 et une solution de détection contenant au moins une première amorce.

41. Kit selon la revendication 40, dans lequel la solution de détection contient une seconde amorce.
